# EUROPÄISCHE PATENTANMELDUNG

(11) **EP 1 306 063 A1**
(43) Veröffentlichungstag der Anmeldung: **02.05.2003**
(21) Anmeldenummer: 02020101.8
(22) Anmeldetag: 07.09.2002
(51) Int. Cl.: A61F 2/06

(54) **Stent**

(30) Priorität: 25.10.2001 DE 10152066
(71) Anmelder: Curative AG, 01307 Dresden (DE)
(72) Erfinder: Friedrich, Holger, Dr., 01277 Dresden (DE); Schneider, Detlef, 58135 Hagen (DE)
(74) Vertreter: Kailuweit, Frank, Dr. Dipl.-Ing.

(57) **Zusammenfassung**

Die Erfindung betrifft auf dem Gebiet der Behandlung von Gefäßverengungen einen Stent für den Einsatz in ostiumnahen Bereichen arterieller Gefäßsysteme. Der Stent umfasst einen rohrförmigen Spreizkörper aus einem Gittergewebe (1) zum Einsetzen in den Ein- oder Abgang eines Gefäßes, wobei das proximale Ende (4) trompetenförmig aufweitbar ist. Mit dem erfindungsgemäßen Stent ist es auf einfache, schnelle und sichere Art und Weise möglich, ostiumnahe Stenosen in arteriellen Gefäßsystemen so zu behandeln, dass der gesamte stenotisierte Bereich mit einem Stent abgedeckt werden kann.

## Beschreibung

Die Erfindung betrifft einen Stent für die Behandlung von arteriellen Gefäßverengungen im sogenannten Gefäß-Ostium, insbesondere für den Einsatz in den Abzweig der Nierenarterie von der Hauptarterie oder im Hauptstamm des Herzkranzgefäßsystems.

Im Rahmen der sogenannten Arteromatose des arteriellen Gefäßsystems kommt es zur Ablagerung und konsekutiven Verengung von arteromatösem, gemeinhin als Kalk bezeichneten Material in den Gefäßwänden. Diese Ablagerungen führen zu einer zunehmenden Verengung der Gefäße und bewirken einen entsprechenden Elastizitätsverlust sowie gleichzeitig eine Einengung des Lumens. Von dieser Form der Gefäßerkrankung sind unter anderem Herzkranzgefäße und regelmäßig auch die Nierenarterien betroffen. Im Bereich der Nierenarterien kann die Verengung einerseits zur Minderdurchblutung der Nieren und konsekutiv zur Entwicklung eines allgemeinen Bluthochdruckes führen. Daher ist eine aggressive Behandlung der Nierenarterienverengung durch Kalkablagerungen angezeigt. Die Nierenarterien gehen annähernd rechtwinklig aus der Hauptschlagader, der sogenannten Bauchaorta, beiderseits hervor. Die Verengungen betreffen typischerweise das sogenannte Gefäß-Ostium, das heißt, die Verengungen treten unmittelbar an der Abgangsstelle der Nierenarterien von der Hauptschlagader auf. Die bisherige invasive Behandlung besteht einerseits in Erweiterung durch Ballonkatheter und anderseits zur Aufrechterhaltung dieser Erweiterung dem nachfolgenden Einbringen von sogenannten Stents (Gefäßstützen). Stents bestehen meist aus aus Draht gefertigten röhrenförmigen Körbchen, die gegen die Wand des jeweiligen Gefäßes gedrückt werden und dort verbleiben. Somit sollen diese Stents als Schienung und stützende Verschalung des wiedereröffneten Gefäßes dienen. Sie können durch entsprechende Materialwahl ballon- und selbstexpandierbar ausgeführt sein. So ist zum Beispiel in dem Gebrauchsmuster DE 91 17 152 U1 eine perkutane Stentanordnung zum Durchgängighalten von Gefäßen und Verhinderung von Reststenosen beschrieben, die selbstexpandierend ist. Sie besteht aus mehreren am inneren Umfang einer flexiblen Hülse angeordneten, zick-zack-förmigen Stützdrahtringen, die mittels einem angeschweißten axial angeordneten Draht, der die Stützdrahtringe verbindet, auf Abstand gehalten werden. Aufgrund der angeführten anatomischen Besonderheit der Nierenarterienverengung müssen diese Stents auf jeden Fall das Gefäß-Ostium, das heißt, den Gefäßeingang von der Hauptschlagader aus gesehen, überdecken. Deshalb werden derartige Stents zum Beispiel in der Nierenarterie so platziert, dass das hauptschlagaderseitige Ende des Stents etwa einen Zentimeter in die Hauptschlagader hineinragt. Sie bilden dort einen kronenartigen Vorsprung, der spätere Eingriffe, bei dem mit dem Katheter durch diesen Stent hindurch mit einem Katheter zu arbeiten, praktisch unmöglich machen. Ferner werden durch nicht an der Gefäßwand anliegende Teile des Stents Verwirbelungen im Blut erzeugt, die zu Thromben führen können. Auch kann mit solchen Stentanordnung eine Stenose im Ostiumbereich nicht vollständig beseitigt werden, da der hauptschlagaderseitige Bereich des Ostiums frei bleibt.

Nach der WO 01/21095 ist ein Stent zum Einsetzen in ein sich gabelndes Körperlumen bekannt. Der Stent besteht im Wesentlichen aus zwei röhrenförmigen Körpern aus Metallgittergewebe, von denen ein ersterer eine Öffnung aufweist, in welche ein zweiter Stent eingesetzt werden kann. Der erste Stent wird so in das sich gabelnde Lumen eingesetzt, dass dessen Öffnung an der Abzweigung positioniert ist. Der zweite Stent wird in das abzweigende Lumen so eingesetzt, dass sich die Anfangsteile der Stents in dem sich gabelnden Körperlumen überlappen.

Ungünstig wirkt sich bei dieser Stentanordnung aus, dass diese einen hohen Strömungswiderstand aufweist und das Einbringen einer solchen mehrteiligen Stentanordnung höchst kompliziert und damit zeitaufwendig ist. Längere Operationszeiten und erhöhtes Operationsrisiko sind die unerwünschte Folge.

Der Erfindung liegt deshalb die Aufgabe zugrunde, einen Stent zur Behandlung von Stenosen in einem Gefäßabgang sowie eine Vorrichtung zum Einbringen eines solchen zu schaffen, mit dem der stenotisierte Gefäßabschnitt im Gefäß-Ostium sicher abgedeckt werden kann und der gut an den Gefäßwandungen anliegt, um hohe Strömungswiderstände und Verwirbelungen im Blut zu verhindern. Ferner soll der Stent spätere Eingriffe ermöglichen, bei denen mit einem Katheter durch diesen Stent hindurch gearbeitet werden muss.
Insbesondere für den Einsatz bei Verengungen der Nierenarterien am Gefäß-Ostium soll eine Berücksichtigung der anatomischen Besonderheiten möglich sein und gleichzeitig die Belastung des Gewebes auf ein Minimum reduziert werden.

Diese Aufgabe wird erfindungsgemäß mit den Merkmalen des Anspruches 1 gelöst.

Danach weist der aus einem rohrförmigen Spreizkörper bestehende Stent an seinem proximalen Ende im expandierten Zustand eine trompetenförmige Aufweitung auf.

Für eine optimale Abdeckung stenotisierten Gewebes ist es vorteilhaft, die trompetenförmige Aufweitung des Spreizkörpers in einem Winkel α von 45 bis 60° zur Längsachse des Spreizkörpers auszubilden.

Der maximale Durchmesser der trompetenförmigen Aufweitung des Spreizkörpers sollte ferner in expandiertem Zustand an seinem proximalen Ende wenigstens 20% größer als der Durchmesser des rohrförmigen Teiles des Spreizkörpers sein.

Es ist zweckmäßig, den Spreizkörper aus einem Gittergewebe mit sich kreuzenden, spiralförmig verlaufenden Stegen zu bilden.

Zur Verbesserung der Gewebeverträglichkeit ist es günstig, dass das Gittergewebe im expandierten Zustand am proximalen Ende und/oder am distalen Ende des Gittergewebes als Ring ausgebildet ist.

In einer vorteilhaften selbstexpandierenden Variante besteht das Gittergewebe des Spreizkörpers aus elastischem Material oder aus Material mit Memoryeigenschaften.

Beste Ergebnisse sind mit aus Nitinol hergestellten Gittergeweben erzielt worden.

Es ist aber auch möglich, dass der Spreizkörper aus plastisch verformbarem Material besteht, so dass dieser mittels einem in den Spreizkörper einführbaren Ballon expandierbar ist.

Insbesondere bei der Verwendung des Stents im Bereich der Nierenarterie ist es erforderlich, den distalen Teil, d. h. den Teil, der vom Gefäßeingang weiter entfernt liegt, flexibel zu gestalten, damit dieser den dort physiologisch bedingten Lageänderungen des Gefäßsystems besser folgen kann. Erreicht wird diese Flexibilität vorteilhaft dadurch, dass das Gittergewebe in axialer Richtung beginnend mit einem starren Teil am proximalen Ende abwechselnd flexible und starre Bereiche aufweist.

Die Flexibilität von Teilen des Gittergewebes wird dadurch erreicht, dass die starren Bereiche des Gittergewebes durch drei bis höchstens fünf Stege verbunden sind.

Die genaue räumliche Aufteilung zwischen starren und flexiblen Bereichen ist je nach Anwendungsfall frei wählbar.

Mit dem erfindungsgemäßen Stent wird bei der Behandlung von Stenosen im Gefäß-Ostium, insbesondere bei der Erweiterung und Stützung einer verengten Nierenarterie, eine vollständige Bedeckung des stenotisierten Gefäßabschnittes ermöglicht. Der Stent liegt durch die erfindungsgemäße Ausbildung auch im Bereich des Gefäß-Ostiums gut an der Gefäßwand an, so dass hohe Strömungswiderstände und Verwirbelungen im Blut durch von der Gefäßwand abstehende Teile des Stents vermieden werden.

Ferner ergibt sich bei der Anwendung des erfindungsgemäßen Stents der große Vorteil, das spätere Interventionen distal des jeweiligen Ostiums möglich sind.

Nachfolgend wird die Erfindung an einem bevorzugten Ausführungsbeispiel unter Bezugnahme auf die anliegenden Zeichnungen erläutert.

Es zeigen:
- Figur 1:: eine schematische Darstellung des Stents im expandierten Zustand.
- Figur 2:: eine schematische Darstellung des Stents im eingefalteten Zustand.
- Figur 3:: eine schematische Darstellung des in ein Gefäß-Ostium eingesetzten Stent.
- Figur 4:: eine schematische Darstellung des Einführbestecks.
- Figur 5:: eine schematische Schnittdarstellung des distalen Teils des Einführbestecks mit einem Stent, dessen proximaler Abschnitt expandiert ist.
- Figur 6:: eine schematische Schnittdarstellung des distalen Teils des Einführbestecks mit einem Stent, der über die gesamte Länge expandiert ist.

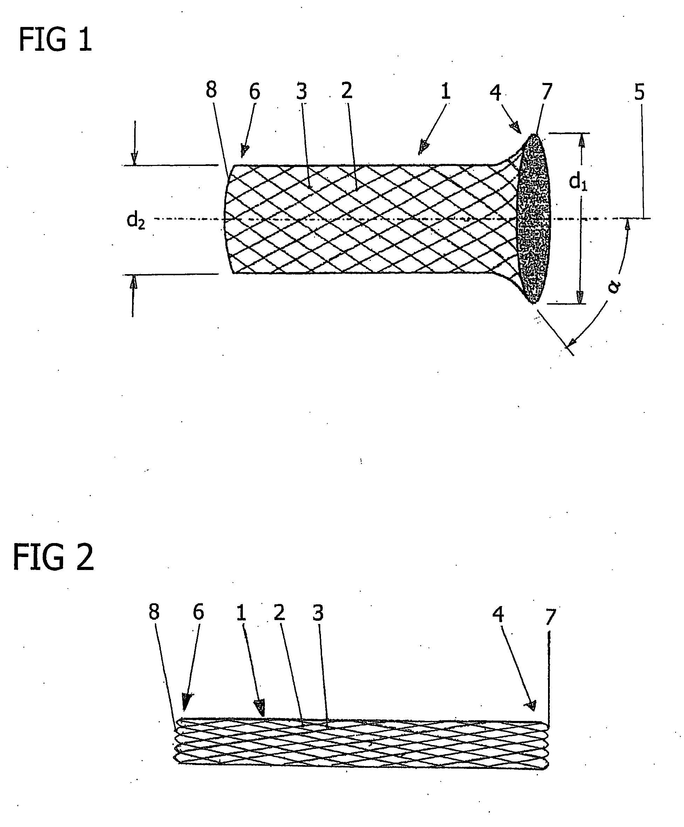

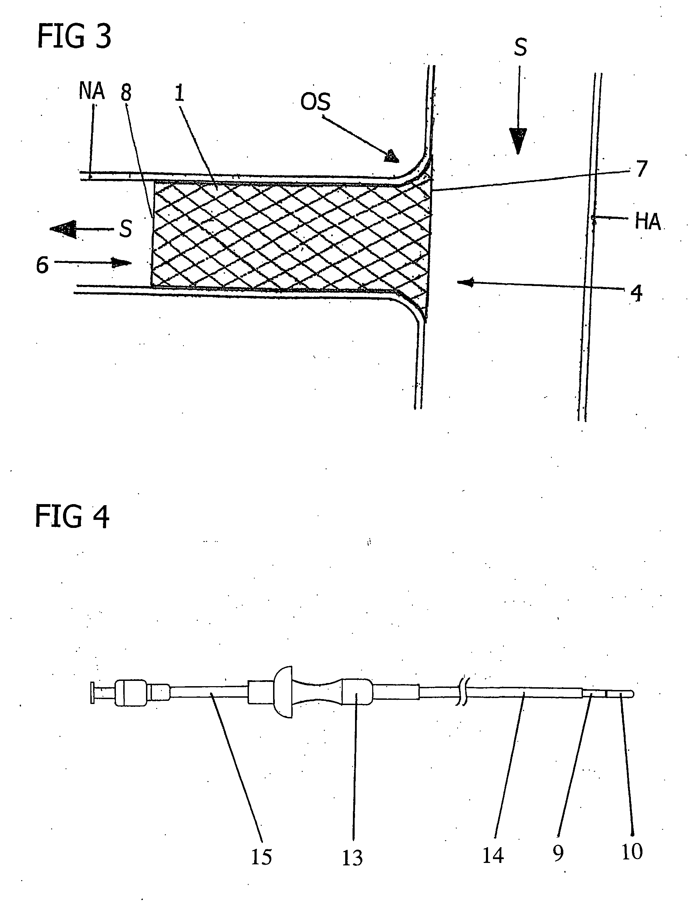

In Figur 1 ist ein erfindungsgemäßer Stent im expandierten und in Fig. 2 im zusammengefalteten Zustand dargestellt. Der Stent besteht aus einem Gittergewebe 1 aus Nitinol, dessen Stege 2 und 3 gegenläufig spiralförmig verlaufen und somit diesem eine rohrförmige Gestalt geben. Am proximalen Ende 4 des Gittergewebes 1 ist dieses trompetenförmig aufgeweitet. Der Winkel α zwischen der Längsachse 5 und der trompetenförmigen Erweiterung beträgt 50°. Der größte Durchmesser d₁ der trompetenförmigen Aufweitung ist 20% größer als der Durchmesser d₂ des übrigen Gittergewebes 1. Am proximalen Ende 4 und am distalen Ende 6 ist das Gittergewebe 1 jeweils mit einem Ring 7, 8 abgeschlossen. Das heißt, die Enden der Stege 2 und 3 sind so miteinander verbunden, dass im expandierten Zustand die Ringe 7 und 8 entstehen. Das Gittergewebe 1 aus Nitinol kann im kalten Zustand, wie in Fig. 2 dargestellt, auf einen sehr kleinen Durchmesser zusammengefaltet werden. Bei Erwärmung auf Körpertemperatur dehnt sich das Gittergewebe 1 dank des Erinnerungsvermögens seines Materials auf seine ursprüngliche in Fig.1 dargestellte Form selbstexpandierend aus. Die dabei auftretenden Kräfte weiten das stenotisierte Gefäß und halten es zuverlässig offen. In Fig. 3 ist der in eine Abzweigung der Nierenarterie NA von der Hauptarterie HA eingebrachte Stent dargestellt. Der trichterförmige, durch den Ring 7 abgeschlossene Teil des Gittergewebes 1 umschließt den Bereich des Ostiums OS der Nierenarterie NA sicher, ohne dass Teile des Gittergewebes 1 in die Hauptarterie HA hineinragen. Mit S ist die Strömungsrichtung des Blutes bezeichnet.

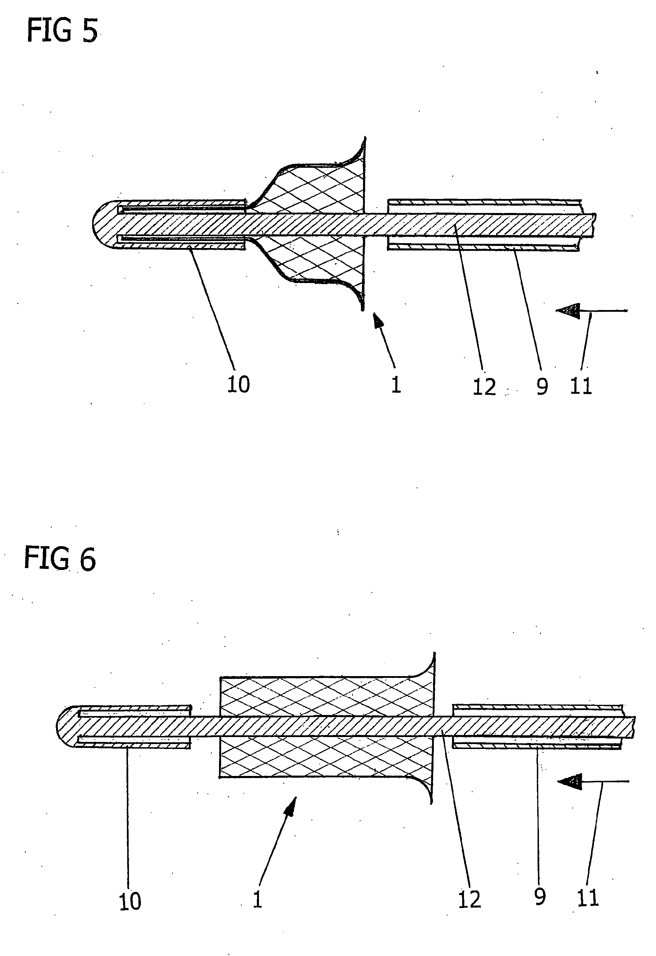

Die Einbringung des Stents in den zu behandelnden Gefäßabschnitt erfolgt in folgender Weise:
Das in Fig. 2 dargestellte zusammengefaltete Gittergewebe 1 wird in zwei hintereinander angeordnete Hülsen, nämlich eine proximale Hülse 9 und eine distale Hülse 10, so eingebracht, dass das proximale Ende 4 des Gittergewebes 1, welches die trompetenförmige Aufweitung aufweist, in der proximalen Hülse 9 und der überstehende Rest in der distalen Hülse 10 zu liegen kommt. Die Hülsen 9 und 10 werden mit Hilfe eines in Fig. 4 dargestellten Einführbestecks in das zu behandelnde Gefäß, z. B. über die Hauptschlagader HA in den Abzweig der Nierenarterie NA eingebracht. Das Einführbesteck weist ein Handhabungsgriff 13 auf, an dem das Futter 14 befestigt ist. Durch den Handhabungsgriff 13 ist der sogenannte Pusher 15 geführt, mit dem die mit diesem verbundene Hülse 9 betätigt werden kann. Innerhalb des Pushers 15 ist eine Seele 12 geführt, die mit der distalen Hülse 10 verbunden ist. Ist der aus dem Gittergewebe 1 bestehende Stent am stenotisierten Abschnitt der Aorta richtig positioniert, wird, wie in Fig. 5 dargestellt, die proximale Hülse 9 vom Gittergewebe 1 entgegen der Richtung des Pfeils 11 abgezogen. Der dabei frei werdende proximale Teil des Gittergewebes 1 mit der trompetenförmigen Aufweitung kann sich damit entfalten. Der distale Teil des Gittergewebes 1 verbleibt zunächst in der distalen Hülse 10. Nachfolgend kann das Gittergewebe 1 in dieser teilentfalteten Form mit dem Pusher 13 über die Hülse 9, in Richtung des Pfeils 11 fest in die Gefäßabzweigung gedrückt werden, so dass die trompetenförmige Erweiterung des Gittergewebes 1 eng am Gefäßostium anliegt.
Anschließend kann über die Seele 12 des Einführbestecks die distale Hülse 10 in Richtung des Pfeils 11 vom Gittergewebe 1 abgezogen werden, so dass sich nun auch der distale Teil des Gittergewebes 1 entfalten kann. Der Stent kommt damit in die in Fig. 3 dargestellte Lage. Durch das entfaltete Gittergewebe 1 kann wie in Fig. 6 dargestellt, nunmehr die distale Hülse 10, die proximale Hülse 9 mit dem gesamten Einführbesteck entgegen der Richtung des Pfeils 11 aus dem Gefäßsystem entfernt werden.

### Bezugszeichenliste:

- 1: Gittergewebe
- 2, 3: Steg
- 4: proximales Ende
- 5: Längsachse
- 6: distales Ende
- 7, 8: Ring
- 9: proximale Hülse
- 10: distale Hülse
- 11: Pfeil
- 12: Seele
- 13: Handhabungsgriff
- 14: Futter
- 15: Pusher
- HA: Hauptschlagader
- NA: Nierenarterie
- OS: Ostium
- S: Strömungsrichtung des Blutes

## Patentansprüche

1. Stent zum Einsetzen in eine Abzweigung eines Gefäßes, insbesondere für die Abzweigung der Nierenarterie von der Hauptarterie, bestehend aus einem rohrförmigen Spreizkörper, der ein proximales Ende (4) und ein distales Ende (6) aufweist, **dadurch gekennzeichnet, dass** das proximale Ende (4) im expandierten Zustand eine trompetenförmige Aufweitung aufweist.

2. Stent nach Anspruch 1, **dadurch gekennzeichnet, dass** die trompetenförmige Aufweitung zur Längsachse (5) des Stents einen Winkel (α) von 45 bis 60° aufweist.

3. Stent nach Anspruch 1 oder 2, **dadurch gekennzeichnet, dass** der größte Durchmesser (d₁) der trompetenförmigen Aufweitung am proximalen Ende (4) wenigstens 20% größer als der Durchmesser (d₂) des rohrförmigen Teiles des Spreizkörpers ist.

4. Stent nach einem der Ansprüche 1 bis 3, **dadurch gekennzeichnet, dass** der Spreizkörper aus einem Gittergewebe (1) mit sich kreuzenden, spiralförmig verlaufenden Stegen (2, 3) besteht.

5. Stent nach Anspruch 4, **dadurch gekennzeichnet, dass** im expandierten Zustand das proximale Ende (4) und/oder das distale Ende (6) des Gittergewebes (1) als Ring (7, 8) ausgebildet ist.

6. Stent nach einem der Ansprüche 4 oder 5, **dadurch gekennzeichnet, dass** das Gittergewebe (1) aus elastischem Material oder Material mit Memoryeigenschaften selbstexpandierend ausgebildet ist.

7. Stent nach Anspruch 6, **dadurch gekennzeichnet, dass** das Gittergewebe (1) aus Nitinol besteht.

8. Stent nach einem der Ansprüche 1 bis 5, **dadurch gekennzeichnet, dass** das Gittergewebe (1) aus plastisch verformbaren Material besteht.

9. Stent nach einem der Ansprüche 1 bis 8, **dadurch gekennzeichnet, dass** das Gittergewebe (1) in Richtung der Längsachse (5) abwechselnd starr und flexibel ausgebildete Bereiche aufweist, wobei das proximale Ende (4) als starrer Bereich ausgebildet ist.

10. Stent nach Anspruch 9, **dadurch gekennzeichnet, dass** die flexiblen Bereiche des Gittergewebes (1) durch drei bis höchstens fünf die starren Bereiche des Gittergewebes (1) verbindende Stege (2, 3) gebildet sind.
